Europäisches Patentamt

European Patent Office

Office européen des brevets

(19)

(11) Publication number: **0 077 119**
**A2**

(12) **EUROPEAN PATENT APPLICATION**

(21) Application number: **82303985.4**

(22) Date of filing: **28.07.82**

(51) Int. Cl.³: **A 61 K 35/16**

---

(30) Priority: **01.10.81 CA 387063**

(43) Date of publication of application: **20.04.83**
**Bulletin 83/16**

(84) Designated Contracting States: **AT BE CH DE FR GB IT LI NL SE**

(71) Applicant: **Rock, Gail Ann, 270 Sandridge Road, Rockcliffe Park Ottawa Ontario K1L 5A2 (CA)**

(72) Inventor: **Rock, Gail Ann, 270 Sandridge Road, Rockcliffe Park Ottawa Ontario K1L 5A2 (CA)**

(74) Representative: **Lawrence, Peter Robin Broughton et al, Gill, Jennings & Every 53-64 Chancery Lane, London WC2A 1HN (GB)**

---

(54) **Collection of antihemophilic factor VIII.**

(57) Blood plasma that has been collected using heparin or sodium heparin (or a mixture thereof) as the sole anticoagulant is frozen, the plasma is resolubilised, a cryoprecipitate obtained from the resolubilised plasma is resolubilised, a saline solution containing heparin is added to the resolubilised cryoprecipitate and the product is incubated to produce a precipitate that is rich in Factor VIII and cold-insoluble globulin, and Factor VIII is isolated from this precipitate.

60/2023/02          1     GAIL ANN ROCK
COLLECTION OF ANTIHEMOPHILIC FACTOR VIII

Various methods are known for collecting concentrates of Factor VIII from blood plasma, which may have been obtained either from whole blood or by plasmapheresis.    The methods generally involve freezing the plasma and obtaining a cryoprecipitate, and optionally other steps.    The blood or blood plasma is initially collected into an anticoagulant, for instance in the manner conventionally used at blood transfusion centres.

Unfortunately it is difficult to obtain a good yield of Factor VIII in the concentrate and it is difficult to obtain concentrates of adequate purity.

In U.S. Patent No. 4,203,891 there is described a method of greatly increasing the yield of antihemophilic Factor VIII (AHF) obtained from whole blood, blood plasma or blood plasma fractions based on the maintenance of physiological concentrations of calcium and/or other ions in the whole blood or plasma components.  In the method described an attempt was made to preserve the initial Factor VIII activity in plasma as much as possible while at the same time to improve the recovery of Factor VIII in cryoprecipitate prepared from that plasma.

In European Patent Publication 0033582 (application 81300069.2) there is described a method of obtaining Factor VIII which involves collection of the blood or plasma into citrate and heparin and also involves the introduction of a cold insoluble globulin (CIg) or fibronectin step to Factor VIII production which results in markedly increased yields of Factor VIII in the cryoprecipitate and, as well, in the cold-insoluble globulin or fibronectin obtained from the cryoprecipitate. As was discussed in the disclosure of this patent, the cold precipitation of fibronectin and fibrinogen had been an established procedure for many years prior to the original filing date in 1972 of the Fekete U.S. Reissue patent 29,698. It was generally known that addition of heparin or some other polysaccharide compound was necessary in order to effect precipitation of fibronectin in the cold. However, at no point had the literature suggested or contained directions for the application of a cold-insoluble globulin or fibronectin purification step toward the production of Factor VIII. In fact, a 1975 paper by Mosher, entitled "Cross Linking of Cold-Insoluble Globulin by Fibrin-Stabilising Factor", published in J.B.C. 250: 6614, 1975, indicated that cold-insoluble globulin was distinguished from antihemophilic factor (Factor VIII) by amino acid analysis, by the position of elution from 4% agarose gels and by the electrophoretic migration of polyacrylamide gels.

Although it is possible to obtain an improvement in yield by this method compared to previous methods the yield and purity of the product could still advantageously be improved. In particular the purity of the concentrate may be rather unsatisfactory for many purposes and so may have to be subjected to other conventional treatments for increasing its purity prior to use. Thus, as mentioned in that specification, the concentrate may be subjected to fractionation by precipitation, by glycine, ethanol, ethanol-glycine, polyethylene glycol or glycine-polyethylene glycol precipitation and/or other known purification agents. This lack of purity of the initial concentrate is a particular problem when the Factor VIII is obtained starting from whole blood.

3

It has been the applicants object to obtain Factor VIII concentrates in good yield and purity.

In the invention a Factor VIII concentrate is obtained by a method comprising freezing blood plasma that has been collected using a first anticoagulant, or a fraction of such blood plasma, resolubilising the plasma, resolubilising a cryoprecipitate obtained from the resolubilised plasma, adding a saline solution of second anticoagulant to the resolubilised cryoprecipitate and incubating the resultant product to obtain a precipitate that is rich in Factor VIII and cold insoluble globulin, both of which are precipitable in the presence of heparin, and isolating Factor VIII from this precipitate, and the method is characterised in that the first anticoagulant consists substantially only of heparin and the second anticoagulant comprises heparin.

The invention is based, at least in part, on the discovery that the cold-insoluble globulin technique can be applied to cryoprecipitates obtained from blood or plasma collected into heparin or sodium heparin or mixtures of the two to obtain a high purity Factor VIII. Thus by the invention it is possible consistently to obtain a high purity product without the need for subsequent purification stages, whereas the process of European publication 0033582 tended to produce a product of lower purity in many instances.

The process of the invention has other advantages and in particular the process of collecting blood or blood plasma into heparin together with the cold incubation step produces a cold insoluble globulin-like material which is very rich in Factor VIII. This new process permits recovery of a high purity Factor VIII preparation which can easily be made in the blood bank or, alternatively, the cold precipitation step can be carried out in a fractionation plant.

4

The method of the invention results in the recovery of high purity Antihemophilic Factor VIII from whole blood or blood plasma or blood plasma fractions. If whole blood is used as the starting material it is freshly collected directly into an anticoagulant selected from heparin, sodium heparin and mixtures thereof and the red cells are separated from the plasma. The amount of heparin is from 2 to 6, preferably 3.5 to 4, units heparin per ml blood, preferably being equivalent to 6 to 8 units, most preferably 8 units heparin per ml plasma. Alternatively the plasma may be obtained by plasmaphoresis using heparin as anticoagulant rather than citrate phosphate dextrose. The amount of heparin used may be from 4 to 10, preferably 6 to 8 and most preferably 8 units heparin per ml plasma. Normally the whole of the plasma is used but if desired the process may be carried out using a blood plasma fraction instead of the whole plasma.

The resultant heparinised plasma is then frozen and this may be conducted by standard blood bank techniques e.g. at about -80°C. The plasma may then be resolubilised, e.g. at a temperature of 0 to 10°C for 30 to 120 minutes, preferably at about 4°C for about 75 minutes. A cryoprecipitate may then be separated from the resolubilised plasma, for instance by centrifuging.

The separated cryoprecipitate may then be resolubilised, for instance at about 37°C for 2 to 5 minutes.

A saline heparin solution may then be added to the resolubilised cryoprecipitate, generally in an amount of 2 to 5 ml per bag. This amount is calculated on the assumption that the process is being conducted in conventional blood bank equipment in which event the cryoprecipitate will be contained in, for instance, a 300 ml satelitte pack bag. Expressed alternatively, the amount of heparin that is added may be about 1 unit, typically 0.8 to 1.2 units, heparin

5

per ml of the solution.      Preferably the amount of heparin saline diluent that is added is 2.5 mls per bag. Although it is less preferable it is possible to use a citrate saline heparin buffer in this step instead of a heparin buffer.      However the use of the citrate saline heparin buffer does lower the yield of Factor VIII.

The resultant product is then incubated at a low temperature, generally 0 to 4°C, for a prolonged period, generally 1 to 6 hours, preferably about 2 hours or more.      The product that is subjected to this incubation includes not only Factor VIII but also cold-insoluble globulin and both of these are precipitated during the incubation in the presence of heparin. Accordingly the precipitate that is obtained by this step includes both Factor VIII and cold insoluble globulin. Generally this incubation step is conducted on a volume of product obtained by pooling several bags of resolubilised cryoprecipitate.

The total precipitate is separated from the supernatent, which is then poor in Factor VIII, generally by centrifuging at a low temperature, typically 0°C.

The Factor VIII is then isolated from the precipitate.      This may be effected by washing the separated precipitate with cold saline heparin solution and resolubilising in the presence of heparin saline. The washing is generally conducted at a temperature of, about 0°C.      The amount of saline heparin solution used for the washing may be from 2 to 4 ml, preferably about 2.5 ml per original cryoprecipitate bag. Resolubilisation of the washed precipitate is best conducted at an elevated temperature, often about 37°C, after adding further heparin saline diluent.      The amount that is added is normally 2 to 10 ml, preferably 2 to 4 ml and most preferably about 2 ml per original cryoprecipitate

6

bag. The mixture of precipitate and saline solution may then be maintained at about 37°C for 5 minutes, to allow solubilisation to occur. The Factor VIII content becomes solubilised and so separation, e.g. by centrifugation, results in removal of a supernatent solution that is rich in Factor VIII.

The method of this invention may be described in precise terms as follows. Fresh heparin plasma at 8 units heparin per ml is frozen to -80°C, then resolubilised at 4°C for 75 minutes, after which it is centrifuged (spin 7000 x g, 7 min at 0°C). The resulting cryoprecipitate is allowed to resolubilise at 37°C for 2-5 minutes, after which 2.5 ml heparin saline buffer, pH 7.2 per cryoprecipitate bag is added. The resolubilised, pooled cryoprecipitate is then incubated at 0°C for two hours in a refrigerated water bath. The Factor VIII present in the cryoprecipitate pool now insolubilises along with the cold-insoluble globulin, and the total insoluble precipitate is then separated from the Factor VIII poor supernatant by centrifugation (spin 7000 x g, 7 min at 0°C). After draining off the supernatant, the cold-insoluble precipitate is washed at 0°C with 2-4 ml of cold saline heparin solution per original bag, drained and allowed to resolubilise at 37°C for five minutes with from 2-10 ml, preferably 2 ml saline heparin solution per original cryoprecipitate bag. A further centrifugation at 22°C (spin 3700 x g, 6 min) is conducted and the Factor VIII rich solution in the supernatant is separated from the sedimented debris.

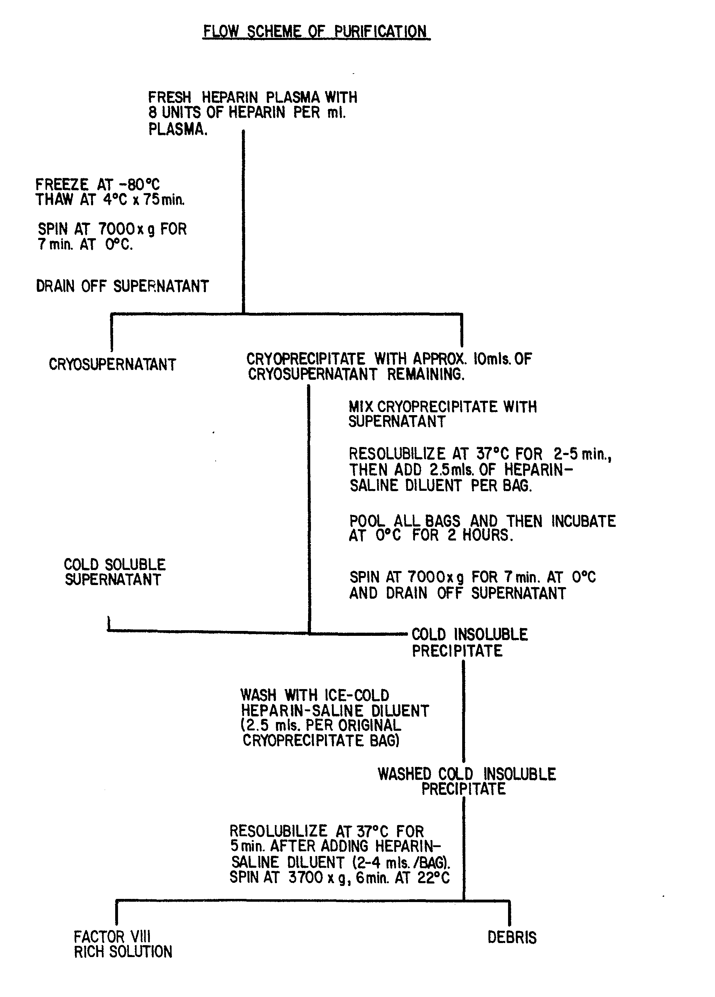

In the drawing which is used to illustrate this invention there is shown a flow sheet which helps to illustrate the specific steps of this procedure as have just been described.

Using this new technique as can be seen from the tables below, it is possible to obtain a high purity, high yield Factor VIII product with recovery of more than 50% of the starting Factor VIII activity and a final yield in the range of 800 units per litre of starting plasma.

In the method of European Patent Publication 0033582, the final product is often an intermediate purity Factor VIII, whereas the present technique always produces a high purity product. This is most advantageous, since it eliminates the need for the addition of chemical precipitants, such as those employed in Cohn fractionation techniques, i.e. ethanol and salts or others such as ammonium sulphate, polyethylene glycol, or polyelectrolytes. In addition, no pH adjustments are required.

The resulting material can be lyophilised or otherwise prepared for storage and infusion.

### TABLE 1

Heparin Plasma Fractionation by the Cold-Insoluble Cryo- precipitate Technique

| Fraction | Total Units of F.VIII:C | Total Protein |
|---|---|---|
| Plasma Pool | 2246 U (100%) | 135,479 mg (100%) |
| Cryoprecipitate Pool[+] litre | 1814 U (81%) | 5,409 mg (4%) |
| Cold-Insoluble Cryoprecipitate | 1193 U (53%) | 1,208 mg (0.9%) |

| Fraction | Specific Activity | Purity Over Plasma | Recovery Per Litre Plasma |
|---|---|---|---|
| Plasma Pool | 0.0166 U per mg | 1 x | 1500 Units per litre |
| Cryoprecipitate Pool[+] Litre | 0.3354 U per mg | 20.2 x | 1211 Units per litre |
| Cold-Insoluble Cryoprecipitate | 0.99 U per mg | 60 x | 797 Units per litre |

[+] resolubilised as required in heparin - saline diluent (heparin at 1 unit per ml, 0.9% NaCl, pH 7.2).

8

TABLE 2

Analysis of Contents

| Fraction | Total Protein | Total Fibrinogen | Total Albumin |
|---|---|---|---|
| Plasma Pool | 135,479 mg (100%) | 2964 mg (100%) | 49,102 mg (100%) |
| Cryoprecipitate Pool | 5,409 mg (4%) | 981 mg (33%) | 2,448 mg (5%) |
| Cold-Insoluble Cryoprecipitate | 1,208 mg (0.9%) | 414 mg (14%) | 132 mg (0.3%) |

| Fraction | Antibody Titre | Volume |
|---|---|---|
| Plasma Pool | 1/128 (100%) | 1497 mls (100%) |
| Cryoprecipitate Pool | 1/128 (6%) | 90 mls (6%) |
| Cold-Insoluble Cryoprecipitate | 1/32 (0.5%) | 27 mls (1.8%) |

The present technique could be carried out in blood bags, that is without the use of diluents. Enough heparin appears to be carried through the whole procedure so that no further additions are required. Thus the technique could be carried out readily in small collection centres.

The advantages of the present invention can be summarised by the following statements.

1. A high purity product can be obtained without the requirement for conventional protein precipitation reagents or without the need for pH adjustment.

2. The procedure is simple and the technology involved is within the capabilities of a small blood bank, yet the procedure is also readily adaptable to large scale fractionation procedures.

3. The procedure can be carried out entirely in the original cryoprecipitate bag, although pooling of several bags prior to the 0°C incubation is preferable.

4.      The final product may be administered immediately, freeze dried or frozen for subsequent administration.   This may be done in the bag itself or the final product may be aseptically transferred to another appropriate container as required.

5.      The method is fast, requiring generally only 2 hours of incubation time at 0°C of the resolubilised plasma cryoprecipitate, with subsequent centrifugation steps being of short duration.

6.      The present method maintains Factor VIII coagulant activity and stabilises Factor VIII during fractionation by maintaining physiological calcium level and a slightly alkaline pH.

7.      The final product may be subjected to additional fractionation by conventional techniques where desired or required.

Typically,   the final product is a high yield, high purity Factor VIII concentrate at 250 units of Factor VIII per 10 ml volume, the protein content is 25 mg per ml, fibrinogen is at 8.7 mg per ml, and the heparin level is 10 units per ml.   The overall recovery is 700 - 800 units per litre of starting plasma. The high yield and purity of the final product enable blood banks to prepare sufficient Factor VIII concentrate for hempohiliacs.   Use of the present technique would provide one million Factor VIII units per 5000 to 6000 donations.

CLAIMS

1.      A method of obtaining Factor VIII comprising freezing blood plasma that has been collected using a first anticoagulant, or a fraction of such blood plasma, resolubilising the plasma, resolubilising a cryoprecipitate obtained from the resolubilised plasma, adding a saline solution of second anticoagulant to the resolubilised cryoprecipitate and incubating the resultant product to obtain a precipitate that is rich in Factor VIII and cold insoluble globulin, both of which are precipitable in the presence of heparin,and isolating Factor VIII from this precipitate, characterised in that the first anticoagulant consists substantially only of heparin and the second anticoagulant comprises heparin.

2.      A method according to claim 1 characterised in that the second anticoagulant consists substantially only of heparin.

3.      A method according to claim 1 or claim 2 characterised in that the heparin is sodium heparin or a mixture of heparin and sodium heparin.

4.      A method according to any preceding claim characterised in that the plasma is obtained by collecting whole blood into heparin and separating the plasma.

5.      A method according to claim 4 characterised in that the blood is collected into 2 to 6, preferably 3.5 to 4, units heparin per ml blood.

6.      A method according to claims 1 to 3 characterised in that the plasma is obtained by plasmapheresis using heparin.

7.      A method according to claim 6 characterised in that the amount of heparin is from 4 to 10, preferably 6 to 8,units heparin per ml plasma.

8.      A method according to any preceding claim characterised in that the incubation is at 0 to 40°C for from 1 to 6, preferably about 2 hours.

9.      A method according to any preceding claim characterised in that the isolation comprises resolubilising the precipitate preferably at 35 to 40°C for from 2 to 10 minutes in the presence of saline heparin and removing the debris.

10.        A method according to claim 1 substantially as described herein.

## FLOW SCHEME OF PURIFICATION

FRESH HEPARIN PLASMA WITH
8 UNITS OF HEPARIN PER ml.
PLASMA.

FREEZE AT −80°C
THAW AT 4°C x 75 min.

SPIN AT 7000 x g FOR
7 min. AT 0°C.

DRAIN OFF SUPERNATANT

CRYOSUPERNATANT

CRYOPRECIPITATE WITH APPROX. 10 mls. OF
CRYOSUPERNATANT REMAINING.

MIX CRYOPRECIPITATE WITH
SUPERNATANT

RESOLUBILIZE AT 37°C FOR 2-5 min.,
THEN ADD 2.5 mls. OF HEPARIN-
SALINE DILUENT PER BAG.

POOL ALL BAGS AND THEN INCUBATE
AT 0°C FOR 2 HOURS.

COLD SOLUBLE
SUPERNATANT

SPIN AT 7000 x g FOR 7 min. AT 0°C
AND DRAIN OFF SUPERNATANT

COLD INSOLUBLE
PRECIPITATE

WASH WITH ICE-COLD
HEPARIN-SALINE DILUENT
(2.5 mls. PER ORIGINAL
CRYOPRECIPITATE BAG)

WASHED COLD INSOLUBLE
PRECIPITATE

RESOLUBILIZE AT 37°C FOR
5 min. AFTER ADDING HEPARIN-
SALINE DILUENT (2-4 mls./BAG).
SPIN AT 3700 x g, 6 min. AT 22°C

FACTOR VIII
RICH SOLUTION

DEBRIS